(19) Europäisches Patentamt

European Patent Office

Office européen des brevets

(11) **EP 1 431 296 A1**

(12) **EUROPEAN PATENT APPLICATION**

(43) Date of publication:
**23.06.2004 Bulletin 2004/26**

(51) Int Cl.[7]: **C07D 307/08**, C07D 307/32,
C07D 315/00

(21) Application number: **03008832.2**

(22) Date of filing: **24.04.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **20.12.2002 US 324961**

(71) Applicant: **EASTMAN CHEMICAL COMPANY
Kingsport, TN 37660 (US)**

(72) Inventors:
• **Beavers, William Anthony
  Longview, Texas 75604 (US)**
• **Ignatchenko, Alexey Victorovitch
  Longview, Texas 75605 (US)**

(74) Representative:
**Wibbelmann, Jobst, Dr., Dipl.-Chem.
Wuesthoff & Wuesthoff,
Patent- und Rechtsanwälte,
Schweigerstrasse 2
81541 München (DE)**

(54) **Process for preparing alpha- and beta-methyl-gamma-butyrolactone and 3-methyltetrahydrofuran**

(57) The present invention pertains to a novel process for preparing alpha- and beta-methyl-gamma-butyrolactones (MeGBL) and/or 3-Methyltetrahydrofuran (MeTHF) from 3-(hydroxymethyl)tetrahydrofuran (HOMeTHF), 3-formyltetrahydrofuran (FTHF) or a mixture thereof by contacting HOMeTHF, FTHF, or a mixture thereof with a catalyst comprising copper on hydrous zirconia under conditions of temperature and pressure conducive to the formation of MeGBL and/or MeTHF. The process may be performed in the presence of an inert atmosphere and/or hydrogen gas. Further, the process may be performed in the presence of a secondary alcohol.

EP 1 431 296 A1

## Description

Field of the Invention

The present invention pertains to a novel process for preparing alpha- and beta-methyl-gamma-butyrolactones (alpha-MeGBL, beta-MeGBL) and/or 3-Methyltetrahydrofuran (MeTHF).

Background of the Invention

Alpha-MeGBL is known to be useful as a solvent and as an intermediate in the manufacture of various materials such as, for example, polyurethanes. In addition, U.S. Patent 5,532,271 discloses that alpha-MeGBL has immunosuppresant and anti-inflammatory activity and U.S. Patents 4,665,091 and 4,851,436 disclose the use of alpha-MeGBL as a starting material for the synthesis of an antihypercholesterolemic compound. Zell, *Helv. Chim Acta,* (1979), 62(2), 474, discloses that (-)-S-alpha-MeGBL can be the central building block in the formation of the side chain of alpha-tocopherol.

JP 06-092,951 discloses the preparation of alpha-MeGBL by the hydroformylation of methyl methacrylate followed by reduction of the intermediate aldehyde to alpha-methyl-gamma-hydroxybutyrate. Jedlinski et al, *J. Org. Chem.*, (1987) 52(2) 4601 disclose the synthesis of alpha-MeGBL via lactone enolates.

MeTHF produced in accordance with the present invention is useful as an industrial solvent and, more importantly, as a monomer in the manufacture of polymers such as elastomers. MeTHF is used extensively as a modifier for plasticizers giving modified glass transition temperatures and broader elastic ranges.

In addition, both alpha- and beta-methyl-gamma-butyrolactones, or a mixture thereof, may be catalytically reduced to produce MeTHF. See, for example US Patent 5,990,324, US Patent 4,006,104, and US Patent 3,969,371, discussing, inter alia, a reduction of unsubstituted gammabutyrolactone to tetrahydrofuran.

Brief Summary of the Invention

The present invention provides a process for producing alpha-MeGBL and beta-MeGBL (together, MeGBL), or its co-production with MeTHF, by contacting HOMeTHF, FTHF, or a mixture thereof, with a catalyst comprising copper deposited on hydrous zirconia. The process may be performed in the presence of an inert atmosphere, hydrogen gas or a mixture thereof. Further, the process is performed under conditions of temperature and pressure conducive to the formation of alpha-MeGBL and beta-MeGBL and/or MeTHF.

In addition, the invention provides a process in which alpha-MeGBL and beta-MeGBL and/or MeTHF are produced by contacting FTHF and/or HOMeTHF with a secondary alcohol in the presence of a catalyst comprising copper deposited on hydrous zirconia. This process may also be performed in the presence of an inert atmosphere, hydrogen gas or a mixture thereof.

Detailed Description of the Invention

The present invention provides a process for producing alpha-MeGBL and beta-MeGBL (together, MeGBL) or MeTHF, or co-production of alpha-MeGBL and beta-MeGBL (together, MeGBL) and MeTHF, by contacting HOMeTHF, FTHF, or a mixture thereof, with a catalyst comprising copper deposited on hydrous zirconia under conditions of temperature and pressure conducive to the formation of MeGBL and/or MeTHF. The process may be performed in the presence of an inert atmosphere or hydrogen gas or a mixture thereof.

In addition, the invention provides a process in which MeGBL and/or MeTHF are produced by contacting FTHF and/or HOMeTHF with a secondary alcohol in the presence of a catalyst comprising copper deposited on hydrous zirconia. This process may also be performed in the presence of an inert atmosphere or hydrogen gas or a mixture thereof.

The HOMeTHF or FTHF starting materials are commercially available, or may be made by processes known to those skilled in the art. See, for example, US Patent 5,840,928 to Satoh et al., which describes a method for making FTHF. Further, the HOMeTHF may be obtained, for example, by contacting FTHF with hydrogen in the presence of a hydrogenation catalyst to convert FTHF to HOMeTHF. See, e.g., copending US Application Serial No. 10/125,664.

The hydrous zirconia used in our novel process may be any of a number of commercially available zirconia catalyst products, which are commonly used as support media for other metals or metal complexes. In the alternative, the hydrous zirconia for use in the present invention may be made by processes known to those of skill in the art.

For example, the hydrous zirconia may be prepared by treating a zirconium salt or other zirconium precursor compound with a base. Any of a number of zirconium salts may serve as starting materials for the catalyst preparation, although the preferred salts are soluble in water. Examples of zirconium precursor compounds, which may be used include zirconium chloride, zirconium bromide, zirconium iodide, zirconium fluoride, zirconyl chloride, zirconyl bromide, zirconyl iodide, zirconyl fluoride, zirconium nitrate, zirconium sulfate, zirconium bicarbonate, zirconium carbonate, zirconium hydroxide, hydrated zirconias, and zirconium containing organometallic or inorganic complexes. A preferred zirconium source is zirconyl chloride or zirconyl nitrate owing to availability and water solubility.

Synthesis of the hydrous zirconia may employ any of the usual methods known to those of skill in the art, including roasting, precipitation, or thermal decomposition. Base catalyzed precipitation of the hydrous zirconia is preferred because of problems with maintaining the proper acidity and water content when using other methods. The base used may be any of a number of commonly known and used base materials, or even the more exotic base materials although there is no particular advantage in the choice of exotic bases since the function of the base is to replace the zirconium counterion with hydroxide or oxide and to control the pH of the environment. Thus, lithium hydroxide, sodium hydroxide, potassium hydroxide, lithium carbonate, sodium carbonate, potassium carbonate, ammonium hydroxide, ammonium carbonate, or any organic amine containing 1 to 20 carbon atoms may be used as the base.

Typically, the zirconia salt precursor would be dissolved in water prior to treatment with the base. The concentration in the water solution is not critical with any amount from 0.01 to 95 weight percent or saturation being suitable, with concentrations of 1 to 50 weight percent and 10-20 weight percent in water being typical. After treating the zirconium salt solution with base to the proper pH (i.e., near neutrality at, for example 5 to 7), the crude hydrous zirconia is precipitated and recovered by filtration or centrifugation. The crude hydrous zirconia is washed thoroughly with, for example, water to remove as much of the original counter ion (i.e., the anion on the starting Zr salt) as possible, thereby avoiding contaminants that could modify the catalytic properties and/or performance. Thus, for example, at least 90 weight percent, preferably at least 99.9 weight percent, of the original counter-ion is removed by washing to produce an active catalyst.

The final treatment to prepare the hydrous zirconia is calcining. Calcination is carried out at a temperature in'the range of 200°C to 600°C with 250°C to 500°C being more preferred, and 300°C to 400°C being most preferred. The calcination conditions determine the residual water content and the surface area of the catalyst, both of which affect the catalytic activity. The calcining time depends on the final properties desired, but ranges of 10 minutes to 100 hours are preferred with 0.5-10 hours more preferred, and 1-3 hours most preferred. The surface area of the hydrous zirconia is 3 to 300 M$^2$/gram, more preferably 30 to 150 M$^2$/gram, and most preferably 40 to 60 M$^2$/gram.

The catalyst for use in the present invention comprises copper deposited on hydrous zirconia; the zirconia portion of which may be prepared as above or purchased from commercially available sources. The catalyst may made by depositing a metal, a metal complex, or a metal salt in a suitable solvent using incipient wetness, evaporation, thermal decomposition, or metal splattering. A preferred method, however, is coprecipitation of solutions of a copper precursor compound and a zirconium salt or precursor compound using a base, such as sodium hydroxide, with techniques (reagent concentrations, neutralizing bases, final pH's, and calcining times and temperatures) such as those described above for preparing the hydrous zirconia. Examples of copper precursor compounds include copper chlorides, bromides, iodides, fluorides, nitrates, sulfates, phosphates, carbonates, bicarbonates, or aliphatic or aromatic carboxylates containing up to 20 carbon atoms. Other metal precursor compounds which may be used include metal complexes with the foregoing counterions or zero valent and complexed with ammonia, aliphatic or aromatic amines containing up to 20 carbon atoms, aliphatic or aromatic phosphines containing up to 20 carbon atoms, aliphatic or aromatic arsines containing up to 20 carbon atoms.

The amount of copper on the hydrous zirconia employed in the process of the present invention can vary substantially depending on the mode of operation and other process variables. Normally the amount of copper will be in the range of 0.001 to 75 element percent, more typically 1 to 25 element percent, based on the total Copper and Zirconia elements in the catalyst. The preferred catalyst comprises 10 to 20 element percent copper on hydrous zirconia.

Prior to their use, the catalysts are reduced with hydrogen to convert any copper oxides into a lower oxidation state, preferably into a zero valent metal. Although not preferred, this pre-reduction treatment may be accomplished in conjunction with the operation of the hydrogenolysis process of the invention. This pre-reduction generally takes place at temperatures between 200°C and the calcining temperature with 250° to 500°C being more preferred and 300° to 400°C most preferred.

The amount of catalyst normally used will give HOMeTHF and/or FTHF: catalyst gram atoms per mole ratio of 0.1:1 to 10,000:1. Lower reactant:catalyst ratios are typical for the autoclave/slurry modes and the higher ratios are typical for the fixed bed systems. The more preferred reactant:catalyst ratio for either case is 0.1:1 to 100:1 with 0.2:1 to 50:1 being most preferred.

The present invention may be carried out in the presence of water, although it is not required. The amount of water used may vary from 1 to 99 parts by weight per part by weight of the HOMeTHF and/or FTHF reactant. More common amounts of water, although not necessarily preferred are 10 to 90 parts by weight per part by weight of the HOMeTHF and/or FTHF reactant with 20 to 80 parts water (same basis) being most common. In addition, while a secondary alcohol may affect the ratio of the products made by the present process, which is described herein, a secondary alcohol may also serve as a solvent. Secondary alcohols, suitable as solvents, are those described herein. As noted below, cyclohexanol and 2-propanol (isopropanol) are preferred when a secondary alcohol is employed.

We have also found that the product mixture that typically results from operating the present invention, MeGBL and MeTHF, may be manipulated by the presence of a secondary alcohol because the alcohol acts as a hydrogen source. For example, when the process is performed using FTHF in the presence of hydrogen and a secondary alcohol, the product mixture tends to favor formation of MeTHF over MeGBL. Conversely, when the process is performed using FTHF in the presence of hydrogen and the subject catalyst, and a secondary alcohol is not employed, the product mix tends to favor MeGBL over MeTHF.

Similarly, although both MeGBL and MeTHF result from the present process when HOMeTHF is used as a starting material, under an inert atmosphere, the product mixture tends to favor formation of MeGBL over MeTHF when a secondary alcohol is not employed. That is, when contacting HOMeTHF with the subject catalyst and a secondary alcohol, under inert atmosphere, the product mixture tends to contain less MeGBL, and more MeTHF, than when performing the process in the absence of a secondary alcohol.

Suitable secondary alcohols for use in the process of the present invention include 2-propanol, 2-butanol, 2-pentanol, 3-pentanol, 3-methyl-2-butanol, 2-hexanol, 3-hexanol, 3-methyl-2-pentanol, 2-methyl-3-pentanol, 1-phenyl-2-propanol, 1,3-diphenyl-2-propanol and cyclohexanol and their derivatives. The secondary alcohols preferably are secondary alkanols or cycloalkanols, i.e., saturated, aliphatic and cycloaliphatic alcohols, containing from 3 to 20 carbon atoms. 2-Propanol (or isopropanol) and cyclohexanol are the most preferred secondary alcohols. Benzylic and allylic alcohols are not suitably because they tend to hydrogenolyze under the reaction conditions.

The process may be carried out under inert atmosphere, such as nitrogen, carbon dioxide, argon, neon gas, and the like. Hydrogen gas may also be used, especially, when starting with FTHF or co-production of additional MeTHF is desired. Under hydrogen atmosphere both the HOMeTHF and/or FTHF reactant and some MeGBL is converted to MeTHF, which is illustrated by examples below. However, in the absence of hydrogen, a higher rate of the feed conversion can be achieved and so the the production rate to alpha-MeGBL and beta-MeGBL is higher.

The process may be carried out at temperatures in the range of 250° to 500°C, preferably 280° to 400°C, and most preferably 300°C to 320°C. The process is carried out using pressures of 1 to 690 bars absolute (15 to 10,012 pounds per square inch gauge — psig; 100 to 69,000 kPa), preferably 10 to 60 bars absolute (1000 to 6,000 kPa), more preferably 15 to 55 bars (1500 to 5500 kPa), and most preferably 40 to 55 bars absolute (4000 to 5500 kPa).

The reaction time required to give satisfactory results will vary significantly depending upon a number of process variables such as process temperature and pressure, type of atmosphere, and the particular catalyst employed, and which product is more desired. The time range normally is from about 10 sec to 10 min residence time with 10 sec to 90 sec being more typical.

As stated herein, the product resulting from operating the present invention typically contains a mixture of alpha-MeGBL and beta-MeGBL and MeTHF. The resulting product may be easily separated by techniques known to those of skill in the art. For example, since alpha-MeGBL and MeTHF have quite different boiling points (200-201° Celsius and 86° Celsius, respectively), they may easily be separated by distillation.

The ratio of alpha-Me to beta-Me isomer of MeGBL formed in carrying out the process of the invention, is normally in the range of 4.0:1 - 5.5:1 depending on, e.g., the conversion and the residence time. We have found that the higher the conversion and the residence time, the more beta-MeGBL isomer formed.

Examples

The processes provided by the present invention are further illustrated by the following examples. All percentages given in the examples are by weight unless specified otherwise. As used herein, the percent conversion of HOMeTHF (or FTHF) is:

$$\frac{\text{Moles HOMeTHF (or FTHF) Converted to All Products}}{\text{Moles HOMeTHF (or FTHF) Fed}} \times 100$$

percent selectivity to a compound is:

$$\frac{\text{Moles HOMeTHF (or FTHF) Converted to a Compound}}{\text{Moles HOMeTHF (or FTHF) Converted to All Products}} \times 100$$

and percent yield of a compound is:

$$\frac{\text{Moles HOMeTHF (or FTHF) Converted to a Compound}}{\text{Moles HOMeTHF (or FTHF) Fed}} \times 100$$

CATALYST PREPARATION

*Preparation of Copper on Zirconia Support Catalyst*

Zirconium sulfate tetrahydrate (106.5g, 0.3 mol) and copper chloride hydrate (4.0 g, 0.03 mol) were dissolved in 1000 ml of water. Aqueous KOH solution (50%) was added dropwise with occasionally stirring until reaching a pH of 9-10. The light blue precipitate was left in the original solution and thus aged for 48 hours. The precipitate was washed with deionized water until no sulfate ion detected with barium chloride and no chloride ion detected with silver nitrate. The resulting solid was dried at room temperature for 5 hours, and at 110°C for 5 hours. It was then calcined at 400°C for 10 hours. The resulting copper on hydrous zirconia catalyst was reduced under hydrogen for 3 hours at 300°C. The calculated copper content was 10 molar percent.

EXAMPLE 1

*Reaction of FTHF with Copper-on-Hydrous Zirconia Catalyst under hydrogen atmosphere in a batch run.*

A 300 ml autoclave was charged with FTHF (10.0g, 0.1mol), water (10.0 g, 0.55 mol), and a catalyst (10.0 g) comprising 10% copper on hydrous zirconia. The autoclave was flushed with hydrogen three times at 3.4 barg (50 psig; 340 kPa) pressure. The initial hydrogen pressure was set at 13.8 barg (200 psig; 1380 kPa). The autoclave was stirred and heated for 2 hours at 150°C and for an additional 2 hours at 280°C. A sample was taken from autoclave and analyzed by GC which showed that the reaction mixture comprised 49.6% alpha-MeGBL and beta-MeGBL, 42.8% HOMeTHF and 5.3% MeTHF. Stirring and heating of the reaction mixture was continued for an additional 2 hours at 300°C and 44.8 barg (650 psig; 4480 kPa) hydrogen pressure. The autoclave was cooled down to ambient temperature and the gas was vented off. The crude mixture was analyzed by GC. The conversion of FTHF was complete. The selectivities to MeGBL and co-products were: mixture of alpha-Me and beta-Me GBL - 64.4%; MeTHF - 14.3%; and HOMeTHF 21.4%.

EXAMPLE 2

*Reaction of FTHF with Copper-on-Hydrous Zirconia Catalyst under hydrogen atmosphere in a batch run.*

This was done similar to Example 1, except that the autoclave was stirred and heated for 2 hours at 150°C and for additional 8 hours at 300°C. The crude mixture was analyzed by GC. The conversion of FTHF was complete. The selectivities to MeGBL and co-products were: alpha-MeGBL - 76.6%; beta-MeGBL - 13.7%; MeTHF - 3.9%; and HOMeTHF 2.1%.

EXAMPLE 3

*Dehydrogenation of HOMe THF with Copper-on-Hydrous* Zirconia *Catalyst under nitrogen in a continuous run.*

A mixture of water 56%, HOMeTHF 43% feed was fed into a one inch tubular reactor filled with Cu/ZrO2 catalyst (130ml catalytic bed) at 37.9 ml/hr rate at 250-370°C and 54.4 bar (800 psi; 5440 kPa) nitrogen pressure. The product was collected after each 3hrs run and analyzed by GC. The selectivity and production rates are shown in Table 1.

Table 1.

| Variables | | | | Yield, % | | | Conversion | Production, g*L$^{-1}$*hr$^{-1}$ | |
|---|---|---|---|---|---|---|---|---|---|
| Temp. Mid. | Temp. Bottom | Gas Flow Ml/min | feed rate ml/hr | 3-Me-GBL | 4-Me-GBL | 3-Me-THF | % | Me-THF | Me-GBL |
| 290 | 346 | 141.6 | 38.1 | 56.9 | 14.2 | 9.3 | 4.23 | 9.6 | 91.3 |
| 290 | 346 | 141.6 | 39.2 | 54.0 | 13.1 | 11.3 | 5.88 | 11.2 | 81.7 |
| 275 | 335 | 141.6 | 38.4 | 53.1 | 11.3 | 12.0 | 11.21 | 12.8 | 81.7 |
| 275 | 335 | 70.8 | 39.0 | 54.5 | 11.7 | 12.0 | 12.17 | 12.8 | 76.9 |
| 285 | 340 | 283.2 | 39.2 | 51.8 | 11.2 | 10.7 | 10.68 | 11.2 | 80.1 |
| 285 | 340 | 471.9 | 39.0 | 52.7 | 11.5 | 8.4 | 12.43 | 8.0 | 76.9 |
| 285 | 360 | 471.9 | 54.0 | 49.4 | 11.0 | 7.2 | 14.06 | 11.2 | 108.9 |
| 250 | 340 | 943.9 | 90.0 | 24.7 | 4.5 | 1.8 | 40.99 | 4.8 | 78.5 |
| 260 | 370 | 943.9 | 90.0 | 25.3 | 4.7 | 2.1 | 47.99 | 4.8 | 92.9 |

EXAMPLE 4

*Reaction of HOMe THF with Copper-on-Hydrous Zirconia Catalyst under hydrogen in a continuous run.*

A mixture of water (55%), HOMeTHF (43.8%) was fed into a one inch tubular reactor filled with Cu/ZrO$_2$ catalyst (130ml catalytic bed) at 35.0-39.4 ml/hr rate at 260-340°C and 54.4 barg (800 psig; 5440 kPa) hydrogen pressure. The product was collected every 3 hrs and analyzed by GC. The selectivity and production rates are shown in the Table 2.

Table 2.

| Variables | | | | Yield, % | | | Conversion | Production g*L$^{-1}$*h$^{-1}$ | |
|---|---|---|---|---|---|---|---|---|---|
| Temp .Mid. | Temp Bottom | Gas Flow ml/min | feed rate ml/hr | 3-Me-GBL | 4-Me-GBL | 3-Me-THF | % | Me-THF | Me-GBL |
| 260 | 310 | 1416 | 35.0 | 11.0 | 4.9 | 10.7 | 54.6 | 11.2 | 17.6 |
| 280 | 310 | 471.9 | 37.2 | 8.8 | 3.6 | 13.0 | 35.9 | 14.4 | 16.0 |
| 313 | 320 | 235.9 | 38.8 | 17.8 | 7.2 | 33.5 | 67.7 | 38.4 | 33.6 |
| 340 | 340 | 141.6 | 39.4 | 26.1 | 10.4 | 40.4 | 84.4 | 46.4 | 49.6 |

EXAMPLE 5

*Reduction of FTHF with Hydrogen, Continuous Run — Copper / Zirconia Catalyst with secondary alcohol*

A mixture of isopropanol (38.73%), water (36.74%), and FTHF (23.81%) was fed into a one inch tubular reactor filled with Cu/ZrO2 catalyst (130ml catalytic bed) at 37.3-39.4 ml/hr rate at 300-344°C and 800 psi hydrogen pressure. The product was collected every 3 hrs and analyzed by GC. The yield and production rates at full conversion are shown in the Table 3.

Table 3.

| Variables | | | | Yield % | | | | Production | |
|---|---|---|---|---|---|---|---|---|---|
| Temp. Mid. | Temp. Bottom | Gas Flow ml/min | feed rate ml/hr | 3-Me-GBL | 4-Me-GBL | 3-Me-THF | HOMe-THF | Me-THF, g/L-hr | Me-GBL, g/L-hr |
| 315 | 300 | 236 | 39.4 | 21.5 | 10.2 | 52.5 | 7.00 | 33.6 | 24.0 |
| 335 | 320 | 236 | 39.1 | 16.7 | 8.2 | 60.0 | 1.62 | 33.6 | 16.0 |
| 344 | 330 | 378 | 37.3 | 6.9 | 3.4 | 67.8 | 0.00 | 36.8 | 6.4 |

EXAMPLE 6

*Reduction of FTHF with Hydrogen, Continuous Run, Repetition of Example 5 — Copper / Zirconia Catalyst and secondary alcohol*

A mixture of isopropanol (21.91%), water (44.81%), FTHF (30.86%) was fed into a one inch tubular reactor filled with Cu/ZrO2 catalyst (130ml catalytic bed) at 37.7-38.8 ml/hr rate at 310-348°C and 800 psi hydrogen pressure. The product was collected every 3hrs and analyzed by GC. The selectivity and production rates are shown in the Table 4.

Table 4.

| Variables | | | | Yield | | | | Production | |
|---|---|---|---|---|---|---|---|---|---|
| Temp. Mid. | Temp. Bottom | Gas Flow ml/min | feed rate ml/hr | 3-Me-GBL | 4-Me-GBL | 3-Me-THF | HOMe-TH F | Me-THF, g/L-hr | Me-GBL, g/L-hr |
| 348 | 330 | 378 | 38.4 | 8.1 | 4.0 | 55.8 | 0.00 | 43.2 | 11.2 |
| 335 | 320 | 472 | 38.8 | 19.5 | 8.5 | 65.5 | 0.00 | 59.3 | 28.8 |
| 320 | 310 | 944 | 37.7 | 19.8 | 7.6 | 65.7 | 4.23 | 52.9 | 25.6 |

EXAMPLE 7

*Reduction of FTHF with Hydrogen, Continuous Run, Repetition of Example 5 — Copper / Zirconia Catalyst and secondary alcohol*

A mixture of isopropanol (10.91%), water (50.88%), FTHF (35.2%) was fed into a one inch tubular reactor filled with Cu/ZrO2 catalyst (130ml catalytic bed) at 39.4 ml/hr rate at 310-320°C and 800 psi hydrogen pressure. The product was collected after 3hrs run and analyzed by GC. The selectivity and production rates are shown in the Table 5.

Table 5.

| Variables | | | | Yield, % | | | | Production | |
|---|---|---|---|---|---|---|---|---|---|
| Temp .Mid. | Temp. Bottom | Gas Flow ml/min | feed rate ml/hr | 3-Me-GBL | 4-Me-GBL | 3-Me-THF | HOMe-THF | Me-THF, g/L-hr | Me-GBL, g/L-hr |
| 320 | 310 | 944 | 39.4 | 19.5 | 7.4 | 57.8 | 7.62 | 60.9 | 33.6 |

<u>EXAMPLE 8</u>

*Reduction of HOMe-THF, Continuous Run — Copper / Zirconia Catalyst with secondary alcohol*

A mixture of isopropanol 40.52%, water 33.98%, HOMeTHF 25.49% was fed into a one inch tubular reactor filled with Cu/ZrO2 catalyst (130ml catalytic bed) at 37.6-37.9 ml/hr rate at 284-340°C and 800 psi nitrogen pressure. The product was collected every 3hrs and analyzed by GC. The selectivity and production rates are shown in the Table 6.

Table 6.

| Variables | | | | Yield, % | | | | Production | |
|---|---|---|---|---|---|---|---|---|---|
| Temp . .Mid. | Temp Bottom | Gas Flow ml/min | feed rate ml/hr | 3-Me-GBL | 4-Me-GBL | 3-Me-THF | HOMe-THF conversion, % | Me-THF, g/L-hr | Me-GBL, g/L-hr |
| 284 | 300 | 47 | 37.9 | 21.8 | 8.3 | 23.5 | 67.0 | 14.4 | 20.8 |
| 340 | 340 | 47 | 37.6 | 33.9 | 12.7 | 30.6 | 89.0 | 19.2 | 33.6 |

EXAMPLE 9

*Reduction of FTHF, Continuous Run — Copper / Zirconia Catalyst and secondary alcohol*

A mixture of isopropanol (38.73%), water 36.74%, FTHF (23.81%) was fed into a one inch tubular reactor filled with Cu/ZrO2 catalyst (130ml catalytic bed) at 37.9 ml/hr rate at 340°C and 800 psi nitrogen pressure. The product was collected after 3hrs and analyzed by GC. The selectivity and production rates are shown in the Table 7.

Table 7.

| Variables | | | | Yield | | | | Production | |
|---|---|---|---|---|---|---|---|---|---|
| Temp .Mid. | Temp. Bottom | Gas Flow SCF/hr | feed rate ml/hr | 3-Me-GBL | 4-Me-GBL | 3-M-THF | HOMe-THF | Me-THF, g/L-hr | Me-GBL, g/L-hr |
| 340 | 340 | 47 | 37.9 | 25.0 | 8.9 | 27.5 | 0.00 | 14.4 | 22.4 |

**Claims**

1.  Process for preparing a mixture of alpha- and beta-methyl-gamma-butyrolactones (MeGBL) and 3-methyltetrahydrofuran (MeTHF), which comprises contacting 3-formyltetrahydrofuran (FTHF) or 3-hydroxymethyltetrahydrofuran (HOMeTHF), or a mixture thereof, with hydrogen in a reaction zone in the presence of a catalyst comprising copper on hydrous zirconia under conditions of temperature and pressure conducive to the formation of MeGBL and MeTHF.

2.  Process according to Claim 1 wherein the process is carried out at a temperature of 250° to 320°C and a pressure of 15 to 55 bars (1500 to 5500 kPa).

3.  Process according to Claim 1 wherein contacting is carried out in the presence of a secondary alcohol.

4.  Process according to Claim 3 wherein the secondary alcohol is cyclohexanol or 2-propanol.

5.  Process according to claim 4 wherein the process is carried out at a temperature of 250° to 320°C and a pressure of 15 to 55 bars (1500 to 5500 kPa).

6.  Process for preparing a mixture of alpha- and beta-MeGBL and MeTHF, which comprises contacting FTHF or HOMeTHF, or a mixture thereof, with a catalyst comprising copper on hydrous zirconia and, optionally, a secondary alcohol, in a reaction zone under an inert atmosphere and under conditions of temperature and pressure conducive to the formation of MeGBL and MeTHF.

7.  Process according to Claim 6 wherein the inert atmosphere is nitrogen or carbon dioxide.

8.  Process according to Claim 7 wherein the process is carried out at a temperature of 250° to 320°C and a pressure of 15 to 55 bars (1500 to 5500 kPa).

9.  Process according to claim 8 wherein the contacting is carried out in the presence of hydrogen.

10. Process according to claim 7 or 9 wherein the secondary alcohol is cyclohexanol or 2-propanol.

11. Process for preparing a mixture of alpha- and beta- MeGBL, which comprises contacting FTHF with hydrogen in a reaction zone in the presence of a catalyst comprising copper on hydrous zirconia under conditions of temperature and pressure conducive to the formation of MeGBL.

12. Process according to Claim 11 wherein the process is carried out at a temperature of 250° to 320°C and a pressure of 15 to 55 bars (1500 to 5500 kPa).

13. Process for preparing MeTHF, which comprises contacting FTHF with hydrogen and a secondary alcohol in a reaction zone in the presence of a catalyst comprising copper on hydrous zirconia under conditions of temperature and pressure conducive to the formation of MeTHF.

14. Process according to Claim 13 wherein the process is carried out at a temperature of 250° to 320°C and a pressure of 15 to 55 bars (1500 to 5500 kPa).

15. Process according to Claim 13 wherein the secondary alcohol is cyclohexanol or 2-propanol.

16. Process for preparing a mixture of alpha- and beta- MeGBL, which comprises contacting HOMeTHF with a catalyst comprising copper on hydrous zirconia in a reaction zone under an inert atmosphere and under conditions of temperature and pressure conducive to the formation of MeGBL.

17. Process according to Claim 16 wherein the process is carried out at a temperature of 250° to 320°C and a pressure of 15 to 55 bars (1500 to 5500 kPa).

18. Process according to Claim 16 wherein the inert atmosphere is nitrogen or carbon dioxide.

19. Process for preparing MeTHF, which comprises contacting HOMeTHF with a secondary alcohol in a reaction zone under an inert atmosphere in the presence of a catalyst comprising copper on hydrous zirconia under conditions of temperature and pressure conducive to the formation of MeTHF.

**20.** Process according to Claim 19 wherein the process is carried out at a temperature of 250° to 320°C and a pressure of 15 to 55 bars (1500 to 5500 kPa).

**21.** Process according to Claim 19 wherein the secondary alcohol is cyclohexanol or 2-propanol.

**22.** Process according to Claim 21 wherein the inert atmosphere is nitrogen or carbon dioxide:

## EUROPEAN SEARCH REPORT

**European Patent Office**

**Application Number**

EP 03 00 8832

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 6 147 233 A (BEAVERS WILLIAM ANTHONY) 14 November 2000 (2000-11-14)<br><br>* column 3 - column 7; claims *<br>--- | 1-10, 13-15, 19-22 | C07D307/08<br>C07D307/32<br>C07D315/00 |
| X | US 5 912 364 A (BEAVERS WILLIAM ANTHONY) 15 June 1999 (1999-06-15)<br>* claims; examples *<br>--- | 13-15 | |
| A | WO 98 17657 A (EASTMAN CHEM CO) 30 April 1998 (1998-04-30)<br>* claims *<br>--- | 1-22 | |
| A | EP 0 277 562 A (DU PONT) 10 August 1988 (1988-08-10)<br>* column 1 - column 2 *<br>--- | 1-22 | |
| A | CASTIGLION G L ET AL: "VAPOUR PHASE HYDROGENATION OF MALEIC ANHYDRIDE TO UPSILON-BUTYROLACTONE 3.REACTION PATHWAY AND NEW CATALYST COMPOSITIONS" ERDOEL ERDGAS KOHLE, URBAN VERLAG, DE, vol. 48, no. 4/5, 1 April 1995 (1995-04-01), pages 174-178, XP000513420 ISSN: 0179-3187<br>* page 174 - page 175 *<br>--- | 1-22 | **TECHNICAL FIELDS SEARCHED (Int.Cl.7)**<br><br>C07D |
| A | DATABASE WPI Derwent Publications Ltd., London, GB; AN 1995-019255 XP002251309 & JP 06 306069 A (TOSOH CORP.), 1 November 1994 (1994-11-01)<br>* abstract *<br>--- | 1-12, 16-18 | |

-/--

The present search report has been drawn up for all claims

| Place of search | Date of completion of the search | Examiner |
|---|---|---|
| MUNICH | 14 August 2003 | Fazzi, R |

**European Patent Office**

**EUROPEAN SEARCH REPORT**

Application Number

EP 03 00 8832

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DATABASE WPI<br>Derwent Publications Ltd., London, GB;<br>AN 1995-041264<br>XP002251310<br>& JP 06 321925 A (TOSOH CORP.),<br>22 November 1994 (1994-11-22)<br>* abstract *<br>----- | 1-12,<br>16-18 | |
| | | | TECHNICAL FIELDS SEARCHED (Int.Cl.7) |

The present search report has been drawn up for all claims

| Place of search | Date of completion of the search | Examiner |
|---|---|---|
| MUNICH | 14 August 2003 | Fazzi, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

22

# EP 1 431 296 A1

European Patent
Office

**Application Number**

EP 03 00 8832

| **CLAIMS INCURRING FEES** |
| --- |

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

| **LACK OF UNITY OF INVENTION** |
| --- |

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

23

| | | |
|---|---|---|
| **European Patent Office** | **LACK OF UNITY OF INVENTION SHEET B** | **Application Number** EP 03 00 8832 |

The Search Division considers that the present European patent application does not comply with the requirements of unity of invention and relates to several inventions or groups of inventions, namely:

1. Claims: 1-5

   Process for preparing a mixture of alpha- and beta-methyl-gammabutyrolactones (MeGBL) and 3-methyltetrahydrofuran (MeTHF), which comprises contacting 3-formyltetrahydrofuran (FTHF) or 3-hydroxymethyltetrahydrofuran (HOMeTHF) with hydrogen in the presence of a catalyst comprising copper on hydrous zirconia.

2. Claims: 6-10

   Process for preparing a mixture of alpha- and beta-MeGBL and MeTHF, which comprises contacting FTHF or HOMeTHF with a catalyst comprising copper on hydrous zirconia and optionally a secondary alcohol under inert atmosphere.

3. Claims: 11-12

   Process for preparing a mixture of alpha- and beta-MeGBL, which comprises contacting FTHF with hydrogen in the presence of a catalyst comprising copper on hydrous zirconia.

4. Claims: 13-15

   Process for preparing MeTHF, which comprises contacting FTHF with hydrogen and a secondary alcohol in the presence of a catalyst comprising copper on hydrous zirconia.

5. Claims: 16-18

   Process for preparing a mixture of alpha- and beta-MeGBL, which comprises contacting HOMeTHF with a catalyst comprising copper on hydrous zirconia under inert atmosphere.

6. Claims: 19-22

   Process for preparing MeTHF, which comprises contacting HOMeTHF with a secondary alcohol under inert atmosphere in the presence of a catalyst comprising copper on hydrous zirconia.

From what stated above it appears that the only feature linking the six independent claims is the use of the catalyst comprising copper on hydrous zirconia. However, these kind of catalysts are known and moreover from the state of the art (cf. abstacts of JP6306069 and of JP6321925) it is known to use catalysts comprising metals carried on zirconia in order to prepare lactones such as gamma-butyrolactones.

Since the only linking concept between the independent claims of the present application is known and the reagents and products of said

**European Patent**
**Office**

**LACK OF UNITY OF INVENTION**
**SHEET B**

**Application Number**

EP 03 00 8832

The Search Division considers that the present European patent application does not comply with the requirements of unity of invention and relates to several inventions or groups of inventions, namely:

claims are also known and are varied according to the different scope of each claim, it appears that there is no linking concept between the above-mentioned claims.

Hence, in the absence of a single general inventive concept linking claims 1, 6, 11, 13, 16 and 19, involving the same or equivalent special technical features and resulting in a teaching over the prior art, the Search Examiner considers that six separate inventions are to be seen in the present application, namely:

Invention (A): the process for producing alpha- and beta-MeGBL and MeTHF according to claim 1;

Invention (B): the process for preparing a mixture of alpha- and beta-MeGBL and MeTHF according to claim 6;

Invention (C): the process for preparing a mixture of alpha- and beta-MeGBL according to claim 11;

Invention (D): the process for preparing MeTHF according to claim 13;

Invention (E): the process for preparing a mixture of alpha- and beta-MeGBL according to claim 16;

Invention (F): the process for preparing MeTHF according to claim 19.

Since the six concepts have no inventive feature in common, the claims on file comprise six inventions which must be considered non-unitary.

The Applicant is asked to state upon which invention further prosecution of the application should be based and to limit the application accordingly. The other inventions are to be excised from the claims, description and drawings, if any.
The subject-matter to be excised may be made the subject of one or more divisional applications.

EP 1 431 296 A1

## ANNEX TO THE EUROPEAN SEARCH REPORT
## ON EUROPEAN PATENT APPLICATION NO.

EP 03 00 8832

This annex lists the patent family members relating to the patent documents cited in the above-mentioned European search report.
The members are as contained in the European Patent Office EDP file on
The European Patent Office is in no way liable for these particulars which are merely given for the purpose of information.

14-08-2003

| Patent document cited in search report | | Publication date | Patent family member(s) | | Publication date |
|---|---|---|---|---|---|
| US 6147233 | A | 14-11-2000 | NONE | | |
| US 5912364 | A | 15-06-1999 | NONE | | |
| WO 9817657 | A | 30-04-1998 | EP | 0932607 A2 | 04-08-1999 |
| | | | JP | 2002515888 T | 28-05-2002 |
| | | | KR | 2000052661 A | 25-08-2000 |
| | | | WO | 9817657 A2 | 30-04-1998 |
| | | | US | 5990313 A | 23-11-1999 |
| | | | US | 5856527 A | 05-01-1999 |
| EP 0277562 | A | 10-08-1988 | US | 4772729 A | 20-09-1988 |
| | | | DE | 3870635 D1 | 11-06-1992 |
| | | | EP | 0277562 A2 | 10-08-1988 |
| | | | JP | 2543929 B2 | 16-10-1996 |
| | | | JP | 63218669 A | 12-09-1988 |
| JP 6306069 | A | 01-11-1994 | JP | 3194808 B2 | 06-08-2001 |
| JP 6321925 | A | 22-11-1994 | NONE | | |

EPO FORM P0459

For more details about this annex : see Official Journal of the European Patent Office, No. 12/82

26